# EUROPEAN PATENT APPLICATION

(11) **EP 1 113 268 A1**
(43) Date of publication of application: **04.07.2001**
(21) Application number: 99830831.6
(22) Date of filing: 31.12.1999
(51) Int. Cl.: G01N 33/00

(54) **Method and apparatus for air quality monitoring in a predetermined geographical area**

(71) Applicant: Moretti & Carpita S.n.c., 56121 Pisa (IT)
(72) Inventor: Carpita, Angelo, 56122 Pisa PI (IT)
(74) Representative: Celestino, Marco

(57) **Abstract**

A method and apparatus for air quality monitoring in a predetermined geographical area comprising a remote fixed station (1) and at least a mobile station (2). Each mobile station (2) is arranged on a vehicle (3) and comprises selective sensors (4) of polluting gas present in the air, a satellite positioning unit (5) for determining in real time the position of the vehicle (3), a data acquisition and processing unit (6) that receives the signals (7a) from the sensors (4) and the signals (7b) from the positioning unit (5), an interface (8) of transmission of the acquired and pre-processed data (9). The remote fixed station (1) comprises a PC (10) with a modem/fax and antenna board (10a) and program means residing in the PC for displaying and analysing the data. The apparatus allows calculus, such as mapping the average concentration (hourly, daily, monthly, etc.) of the pollutants relatively to the chosen area, in order to obtain a continuous and effective monitoring useful for environmental protection.

## Description

### Field of the invention

The present invention relates to the field of the environmental protection and more precisely it relates to a method for air quality monitoring in a predetermined geographical area.

Furthermore, it relates to an apparatus that carries out this method.

### Description of the prior art

Environmental pollution is considered one of the main cause of respiratory diseases, including cancer.

Excessive concentration of pollutants have often led to limit traffic in cities, to the imposition of certain types of fuel, filters and muffler or to designing changes in city planning or in traffic layout. Monitoring the level of air pollution is therefore very important to improve life quality.

Fixed monitoring units analysing pollution exist used by public environmental offices. However, they have the drawback of not allowing a continuous control on the territory, and the data on pollution that they track are extrapolated for the zone of the city wherein they are not provided.

Movable monitoring units exist as well for detecting pollution, but have the drawback of being very expensive, since they require a continuous presence of workers, and have on board both the sensors of pollutants and the whole apparatus for computing and analysing the sensed data. For covering a large city many movable monitoring units are necessary that work continuously in the traffic. Such units, moreover, give in any case results that do not follow the actual state of pollution, but draw a picture limited of the same in a few predetermined instants. A same area of the city is checked for example once a week or a month, without delivering information of the concentration of pollutants in all the rest of the time and often forgetting the suburbs and extra-urban areas, where often the conditions of pollution are not better than in the town centre.

### Summary of the invention

It is therefore object of the present invention to provide a method for air monitoring that is completely automatic and capable of providing in real time the concentration of pollutants, such as for example CO, NOₓ, S02 and 03, benzene, with a corresponding geographic mapping, on city and extra-urban roads.

It is another object of the invention to provide an apparatus that carries out this method and that has reduced size and minimum impact on a vehicle.

According to a first aspect of the invention, these and other objects are achieved by a method for air quality monitoring in a predetermined geographical area comprising the steps of:
- using at least a vehicle in movement through the area and a remote fixed data processing station;
- arranging on board of the vehicle sensor means for selected pollutants;
- detection of signals proportional to the concentration in the air of the pollutants;
- determination of the instant coordinates of the position reached by the vehicle in the geographical area;
- storage of a set of instantaneous data of the concentration associated to the coordinates;
- transmission in real time of the set of data to the remote fixed data processing station;
- calculus at the remote fixed station of the succession of set of data received about the concentration of pollutants in correlation with the instant positions reached in the geographical area by the vehicle;
- comparison of the data on concentration of pollutants with the reference alarm values of pollution stored in the remote fixed station and display of the calculated data.

Preferably, the step of determination of the instant coordinates of the position reached by the vehicle in the geographical area provides a satellite positioning system of the type GPS, whereas the step of transmission of the set of data is carried out by means of a GSM modem.

Advantageously, the calculus of the data in the remote fixed station provides a mapping of the concentration of pollutants with enhancement of the zones of more concentration where concentration exceeds alarm and/or danger values.

Advantageously, the vehicle on which the mobile station is arranged is a bus, taxi or equivalent public means of transport, whereby the presence of technical workers is not necessary on board.

The sensor means are contained preferably in a shell located on the roof of the vehicle with inlet and outlet openings at the front and the rear faces the shell in the same direction of the speed of the vehicle, said sensor means being surrounded by the air to be monitored owing to the flow that is created between the inlet and the outlet openings due to the speed of the vehicle.

According to another aspect of the invention, an apparatus for air quality monitoring in a predetermined geographical area comprises:
- a mobile station mounted on a vehicle;
- a remote fixed station;
- the mobile station comprising;
- means for fixing the coordinates of the vehicle with respect to a chosen geographical area;
- sensor means for measuring the concentration of selected pollutants in successive locations in the geographical area reached by the vehicle;
- means for associating to sets of instant data the signals proportional to the detected concentration by the sensor means and the signals corresponding to the coordinates of the vehicle;
- means for transmitting to the remote fixed station set of instant data corresponding to the concentration for each position reached by the vehicle;
the remote fixed station comprising:
- means for receiving the set of instant data of the detected concentration and the corresponding coordinates of the vehicle;
- processor means for mapping the geographical area associating the set of detected data on the maps responsive to the successive locations reached by the vehicle.

Advantageously, the mobile station is covered by a shell that has a front cap shape and a rear square side. It is preferably fixed to the roof of the vehicle by means of support shoes that dampen the vibrations of the vehicle.

The gas sensor means, comprise electrochemical detection units for at least one of the following gas: CO, SO₂, NOₓ, O₃, benzene.

### Brief description of the drawings

Further characteristics and/or advantages of the method according to the present invention will be made clearer with the following description of an embodiment thereof, exemplifying but not limitative, with reference to attached drawings wherein:
- figure 1 shows a diagrammatical view of the method for air quality monitoring according to the present invention;
- figure 2 shows a bus on which an apparatus is mounted for air quality monitoring used for carrying out the method according to the invention;
- figures 3 and 4 show a perspective view respectively from the front and the rear of the covering shell of the apparatus mounted on the bus of figure 2, in figure 3 this shell being partially cross sectioned for showing the internal side of the apparatus;
- figure 5 shows a perspective full view of the apparatus partially visible in figure 3;
- figure 6 shows the trend of the air flow crossing the apparatus of figure 3;
- figure 7 shows a detail of a city plan with the corresponding paths of the buses;
- figure 8 shows a city plan comprising the detail of figure 7 with the main roads run by the buses and the intervals of air monitoring;
- figure 9 shows a city plan comprising the detail of figure 7 and of figure 8 with the curve of iso-pollution by CO.

### Description of a preferred embodiment

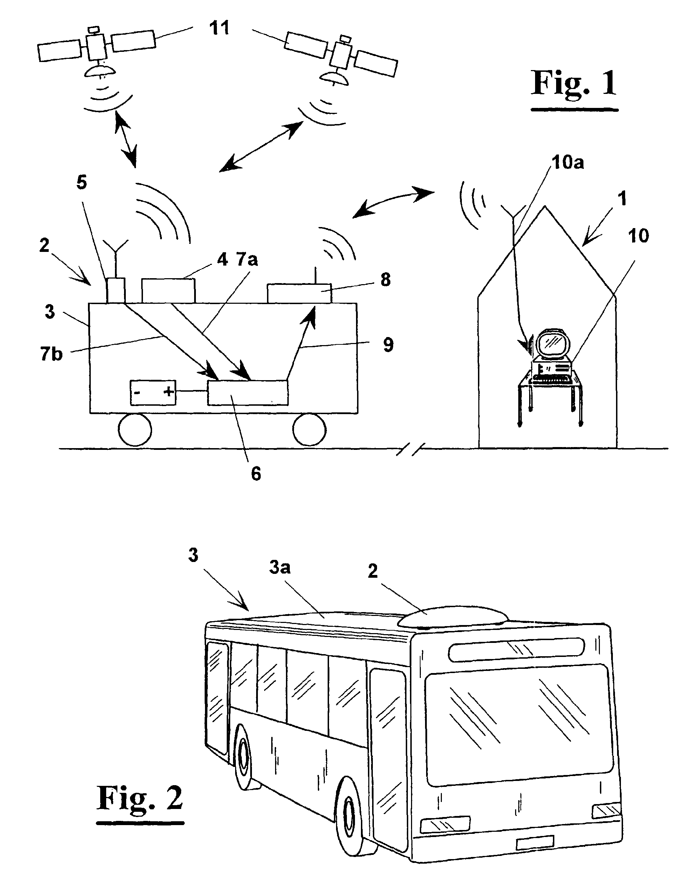

As shown in figure 1, a system that carries out the method according to the invention comprises two stations, a remote fixed station 1 and at least a mobile station 2.

Each mobile station 2 is arranged on a vehicle 3 and comprises:
- selective sensors 4 of polluting gas present in the air,
- satellite positioning unit 5 (GPS) for determining in real time the position of vehicle 3,
- a data acquisition and processing unit 6 that receives the signals 7a from the sensors 4 and the signals 7b from the GPS unit 5;
- a transmission interface 8, for example with the system GSM, of the data acquired and processed 9.

Always with reference to figure 1, the remote fixed station 1 comprises a PC 10 with a modem/fax board and antenna 10a and program means residing in the PC for the display and the analysis of the data.

The mobile station 2 is preferably mounted on a bus in position suitable for having a minimum impact on the vehicle and maximum efficiency of data detection. As shown in figure 2, station 2 is mounted at the front portion of roof 3a of a bus 3 with a front cap shape so that it has:
- minimum impact on the vehicle, both aesthetically, and of minimum encumbrance and aerodynamic drag;
- minima interference with the exhaust gas of the vehicle and good position to receive air not coming directly from the exhaust of the other next vehicles;
- good position of the positioning system 5 and of the interface 8 for data transmission.

As shown in figures from 3 and 4, the mobile station is covered by a shell 12, which has a front cap shape 13 and a rear square side 14. It is fixed to the roof 3a of the vehicle 3 by means of support shoes 15 having dampening effect on the vibrations of the vehicle.

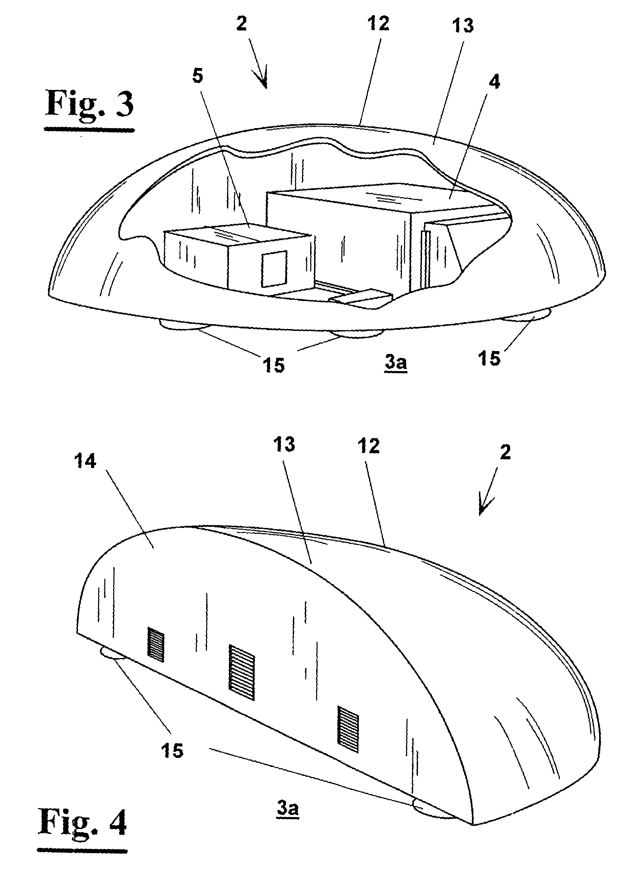

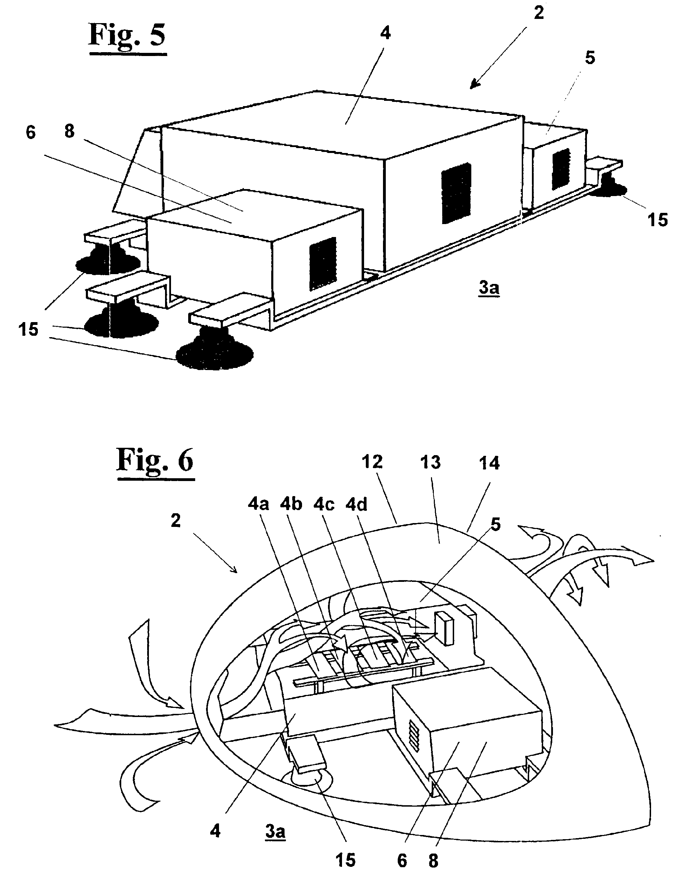

In the shell 12 (fig. 3,5,6) units 4, 5, 6 and 8 are located respectively for analysing the gas, for tracking the position of the vehicle and for calculating and transmitting the data. The flow of the air to analyse is shown in figure 6, indicated by the white arrows and passing at the beginning under cap 13 between shell 12 and roof 3a of the vehicle. Then, the air reaches the gas analyser, and crosses groups 4a, 4b, 4c, 4d, for example for analysis of CO, SO2, NO2, O3 or benzene. Square side 14 of the shell 12, then, with the speed of the vehicle enhances the gas outlet.

Obviously, station 2 can be made in different ways than as above described as an example.

According to the present invention, with reference to figure 1, in a first step of the method the sensing and pre-processing of the data is done at mobile station 2. Gas pollutants sensors 4a-4d are suitable for resisting to vibrations of the vehicle and to different weather conditions and are capable of recording quick changes of the concentration of pollutants through the path of the vehicle.

For example, the sensors are of the electrochemical type (based on electrolytic cells) and transform the variations of the concentration of the polluting gas, such as CO, NO2, SO2 and O3, benzene, into electric signals 7.

In a second step, contemporaneous to the former the positioning of the vehicle is carried out. Preferably, a GPS (Global Positioning System) is used, which determines the location of a vehicle through signals coming from satellites 11. With the systems presently existing the maximum error is of ± 6 m.

Unit 6 calculates the coordinates of the vehicle with respect to a local map, so that data 7 detected on gas pollutants are mapped in real time. Layers are used on roads of the monitored city, the road net, the urban areas, with Gauss Boaga projection. The position of the vehicle provided by the GPS is for example obtained on WGS84 ellipsoid (World Geodetic System 1984). In this case the coordinates are transferred from WGS84 to Gauss Boaga. This is done directly in the vehicle by a software available on the market suitably adapted to geodetic data of the area.

Once data 9 are fixed, they are transmitted by GSM unit 8 to PC 10 on the remote fixed station 1.

In PC 10 a software is residing with a graphic interface suitable also for users not familiar with GIS tools, completely automatic. For example, different windows are shown for every type of pollutant (i.e. CO, NO2, SO2, O3, benzene)

Furthermore, the following are displayed:
- the time of the detection;
- the coordinates (projected into Gauss-Boaga) of the position of the vehicle on wich the data are detected;
- the concentration of the pollutants.

With these information a file of data layers can be created to be added to the windows.

Advantageously, every concentration of pollutant is associated to different layers identified by different colors (fig. 9) for distinguishing the concentration that are in the limits from the concentration that have overcome the alarm limit and the concentration that have overcome the danger limit. Alternatively, iso-pollution lines are shown on the graphic.

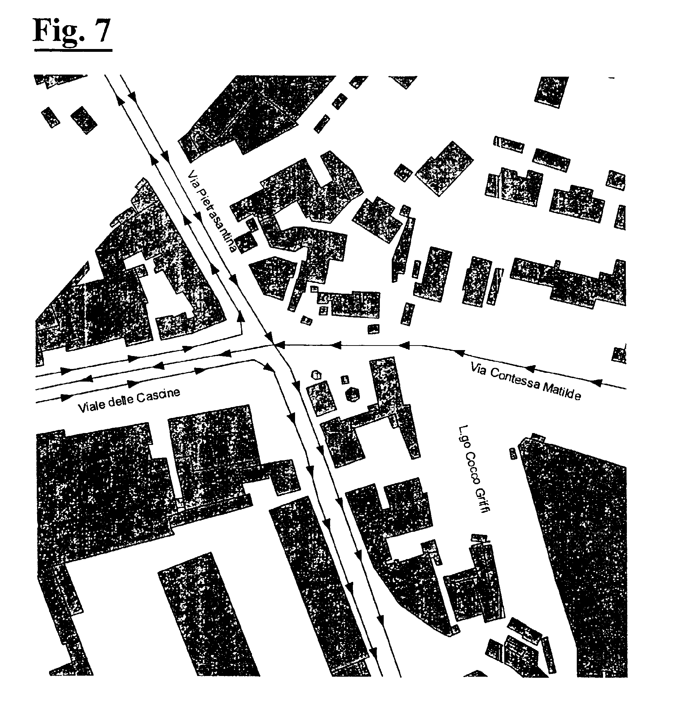

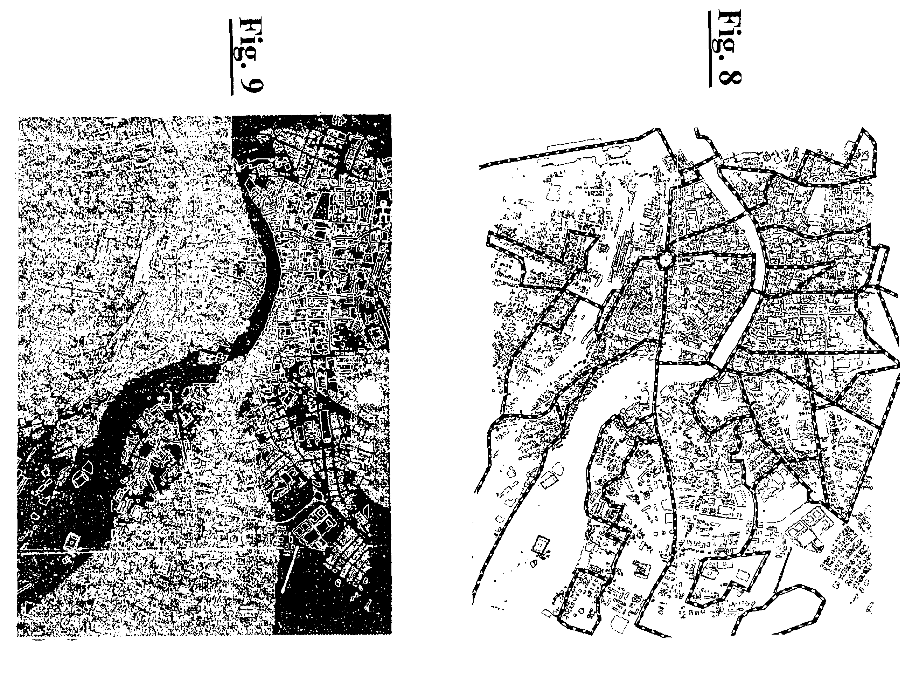

All the graphic layers are added to the windows displaying in real time the points that refer to the location where the detection has been done, the segments indicating the path of the bus, as shown in figure 7 and figure 8. The frequency of detection in station 2 can be predetermined at station 1 according to the paths of the bus. Always as shown in figure 7, the arrows indicate the points of detection and the paths of the bus.

The passage in a same location of the city can also be multiple and contemporaneous. This allows to the remote fixed station to obtain a feedback of the measure of gas pollutants carried out by different mobile stations, with improvement of the quality of measure.

The apparatus allows calculus, for example, of the average concentration (hourly, daily, monthly, etc.) of the pollutants relatively to the monitored area, in order to obtain a continuous and effective mapping of the area.

The availability of the data in electronic format allows the authorities to adopt limitations of the traffic and to change city plans.

Furthermore all the calculated data from the remote fixed station can be easily seen in real time through mass media such as the city nets, multimedia information points and the Internet.

The mobile station 2, which as above said is suitable for being arranged on a vehicle, for example on buses, where the data of pollution and the position of the vehicle are fixed in a way completely automatic, does not require the presence of technical workers on board. This way the costs are dramatically lower than those of presently known mobile stations, and more efficient than the fixed stations present on the territory. The monitoring through several mobile stations allows to the user to check with precision and quickly the areas and the time where and when the concentration of pollutants can overcome the limits of alarm and of danger.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. Method for air quality monitoring in a predetermined geographical area comprising the steps of:
- using at least a vehicle (3) in movement through said area and a remote fixed station (1) of data storage;
- arranging on board of said vehicle sensor means (4) for selected pollutants;
- detection of signals (7a) by said sensors proportional to the concentration of said pollutants;
- determination of the instant coordinates (5) of the position reached by said vehicle (3) in said geographical area;
- storage (6) of a set of instantaneous data (9) of said concentration (7a) associated to said coordinates (7b);
- transmission (8, 10a) in real time of said set of data (9) to said remote fixed station (1) of data storage;
- calculus (10) at said remote fixed station (1) of the succession of set of data (9) received by said vehicle in correlation with said instant positions reached in said geographical area from said vehicle;
- comparison of said set of data received at the various positions reached by said vehicle with reference alarm values of pollution stored in said remote fixed station and recording selectively the positions responsive to said alarm values.

2. Method according to claim 1, wherein said step of determination of the instant coordinates (5) of the position reached by said vehicle (3) in said geographical area provides the use of a satellite positioning system of the type GPS.

3. Method according to claim 1, wherein said step of transmission of said set of data (9) is carried out by means of a GSM modem.

4. Method according to claim 1, wherein said step of calculus provides a mapping of said concentration of said pollutants with enhancement of the zones of concentration exceeding alarm and/or danger values.

5. Method according to claim 1, wherein said vehicle is a bus or equivalent public means of transport, whereby the presence of technical workers is not necessary on board.

6. Method according to claim 1, wherein said sensor means are contained in a shell located on the roof of said vehicle with inlet and outlet openings respectively at the front and the rear of said shell in the same direction of the speed of the vehicle, said sensor means being surrounded by the air to be monitored owing to the flow that is created between said inlet and outlet by the speed of the vehicle.

7. Apparatus for air quality monitoring in a predetermined geographical area characterised in that it comprises:
- a mobile station (2) mounted on a vehicle (3);
- a remote fixed station (1);
said mobile station comprising
- means (5) for fixing the coordinates of said vehicle with respect to said geographical area;
- sensor means (4) for measuring the concentration of selected pollutants in successive locations of said geographical area reached by said vehicle (3);
- means for associating (6) to istant sets of data (9) signals (7a) proportional to the detected concentration by said sensor means (4) and signals (7b) corresponding to said corresponding coordinates;
- means for transmitting (8) to said remote fixed station said instant sets of data (9) corresponding to said concentration for each position reached by said vehicle;
said remote fixed station (1) comprising:
- means for receiving (10a) said instant sets of data (9) of said concentration detected at said vehicle and the corresponding position coordinates;
- processor means (10) for mapping said geographical area associating said sets of detected data on said maps responsive to the successive locations reached by said vehicle.

8. Apparatus according to claim 7, wherein said mobile station is covered by a shell (12) that has a front cap shape (13) and a rear square side (14).

9. Apparatus according to claim 7, wherein said mobile station is fixed to the roof (3a) of the vehicle (3) by means of support shoes (15) having dampening effect on the vibrations of the vehicle.

10. Apparatus according to claim 7, wherein said gas sensor means comprise units (4a, 4b, 4c, 4d) for analysing the electrochemical type of at least one of the following gas: CO, SO₂, NOₓ, O₃, benzene.
